# EUROPÄISCHE PATENTSCHRIFT

(11) **EP 3 090 638 B1**
(45) Veröffentlichungstag und Bekanntmachung des Hinweises auf die Patenterteilung: **06.05.2020**
(21) Anmeldenummer: 15164327.7
(22) Anmeldetag: 20.04.2015
(51) Int. Cl.: A23L 33/155, A61K 33/22, A23L 33/16, A61K 31/593, A61K 33/06

(54) **NAHRUNGSERGÄNZUNG ZUR BEHANDLUNG VON VITAMIN-D3-MANGELERSCHEINUNGEN**
NUTRITIONAL SUPPLEMENT TO TREAT SIGNS OF VITAMIN D3 DEFICIENCY
COMPLÉMENT ALIMENTAIRE DESTINÉ AU TRAITEMENT DE CARENCES EN VITAMINE D3

(43) Veröffentlichungstag der Anmeldung: 09.11.2016
(73) Patentinhaber: Löffler, Bernd-Michael, 10623 Berlin (DE)
(72) Erfinder: Löffler, Bernd-Michael, 10623 Berlin (DE)
(74) Vertreter: Müller & Schubert

(56) Entgegenhaltungen:
- CN-A- 104 415 320
- GB-A- 2 458 467
- US-A1- 2004 162 292
- US-A1- 2006 024 409
- US-A1- 2011 039 809
- "CAL-MAG-BOR-AKTIV und D3-AKTIV 10.000 IE - "ein Bund fürs Leben" bzw. für einen ausgeglichenen Vitamin D3-und Calcium-Magnesium-Haushalt", , 28. November 2014 (2014-11-28), XP055212631, Gefunden im Internet: URL:http://www.omundernaehrung.com/media/d ocuments/NeueProdukteFirmeninfos/2014-11-2 8 CAL-MAG-BOR-AKTIV (ImM-Berlin).pdf [gefunden am 2015-09-10]
- Anonymous: "Neue Produkte und Firmeninformationen - Service - OM & Ernährung", , 10. September 2015 (2015-09-10), XP055212634, Gefunden im Internet: URL:http://www.omundernaehrung.com/service /neue-produkte.html [gefunden am 2015-09-10]
- N.N: "Vitamin D3 / Calcium-Magnesium-Stoffwechsel", , 1. Januar 2015 (2015-01-01), Seiten 7-1430, XP055212655, Gefunden im Internet: URL:http://www.imm.institute/ImM/Seminare_ files/Vitamin D3 _ Calcium Magnesium Stoffwechsel.pdf [gefunden am 2015-09-10]

## Beschreibung

Die vorliegende Erfindung betrifft eine Nahrungsergänzung zur Verwendung in einem Verfahren zur Behandlung von Vitamin-D3-Mangelerscheinungen, umfassend Kalzium, Magnesium und Bor in Kombination mit Vitamin D3 unter Verwendung eines Dosierschemas für die Applikation der Nahrungsergänzung.

Vitamin D3 reguliert den Kalzium-Phosphat Stoffwechsel und somit den Knochenstoffwechsel. Das ist die "klassische Sicht" der Funktion von Vitamin D3, die auch heute noch überwiegt. Seitdem das menschliche Genom 2001 aufgeklärt wurde, und die Molekularbiologie immer größere Bedeutung für das Verständnis der komplexen Zusammenhänge im Stoffwechsel gewinnt, hat sich diese "klassische Sicht" aber grundlegend geändert. Heute ist bekannt, dass Vitamin D3 direkt oder indirekt 3 - 5 % der menschlichen Gene steuert. Das sind mehr als 913 Gene. Der Vitamin D3 Mangel ist nicht nur entscheidend an der Entwicklung chronischer Erkrankungen, einschließlich Herz-kreislauf, Bluthochdruck, Arteriosklerose, Typ-2-Diabetes, beteiligt, sondern der Vitamin D3 Mangel beeinflusst das Leben und die spätere Gesundheit schon vor der Geburt, wenn zum Beispiel die Schwangere während der Schwangerschaft einen Vitamin D3 Mangel hat. Die Bedeutung von Vitamin D3 wird immer noch unterschätzt, und oft zu isoliert betrachtet. Es ist nicht ausreichend bekannt, dass die Einnahme von Vitamin D3 allein nicht hilft, ja sogar kontraproduktiv sein kann.

Kalzium ist mengenmäßig der wichtigster Mineralstoff im Körper des Menschen. Ein Mann hat etwa ein Kilogramm, eine Frau rund 800 Gramm und ein Neugeborenes 30 Gramm davon im Körper. Fast alles, nämlich 99,5 Prozent, ist in Knochen und Zähnen als Kalziumphosphat und Kalziumhydroxyapatit gebunden. Etwa 0,4 Prozent befinden sich in den Zellen. Kalzium-Ionen (Ca²⁺) sind an so wichtigen Funktionen wie der Muskelkontraktion, der elektrischen Nervenleitung und der zellulären Signalvermittlung beteiligt. Nur 0,1 Prozent des Körper-Kalziums findet sich gelöst im Blutplasma. Und davon liegen wiederum nur 47 Prozent, also 0,047 Prozent des Gesamtbestandes, in ionisierter Form als Ca²⁺ vor. Diese Menge entspricht wiederum 1000 bis 1200 mg Kalzium. Das ist die Menge, die täglich (ohne große Belastungsanforderungen) mindestens zugeführt werden muss, um das System im Gleichgewicht zu halten.

Vergleicht man den Körperbestand an Magnesium mit dem von Kalzium, so ist der mit rund 24 Gramm sehr klein. Magnesium ist das 4. häufigste Mineral im Körper und das 2. häufigste in der Zelle. Etwa 65 Prozent davon befinden sich in einem teilweise mobilisierbaren Speicher im Knochen. Magnesium ist ein typisches intrazelluläres Ion (Mg²⁺). Im Blut zirkuliert nur ein kleinerer Anteil des Magnesiums von circa 1 Prozent. Etwa 65 Prozent davon liegt als Mg²⁺-Ion vor. Deshalb werden große Mengen Magnesium über die Niere filtriert. Weil Magnesium eine so herausragende Bedeutung für das Leben besitzt, liegt die Rückresorptionsfähigkeit der Niere für Magnesium bei annähernd 100 Prozent. Im Falle eines Magnesium-Mangels ist der Urin praktisch magnesiumfrei. Das kann unerfreuliche Konsequenzen haben: Schmerzhafte Kalziumoxalat-Nierensteine. Die Bildung dieser Nierensteine hat also nicht nur etwas mit einer Kalzium-Stoffwechselstörung zu tun, sondern vor allem mit Magnesium-Mangel. Deshalb lassen sich Kalziumoxalat Steine in der Niere auch durch die Einnahme von Magnesium auflösen.

Um zum Beispiel den Bedarf von 350 mg Magnesium pro Tag mit der Ernährung zu decken, müsste man so viel essen: 1200 g Fleisch oder 1000 g Fisch oder 200 g Nüsse oder 250 g Haferflocken oder 150 g Haferkeime oder 60 g Weizenkleie oder 75 g Sonnenblumenkerne oder 200 g Bohnen (getrocknet) oder 1200 g Gemüse oder 500 g Spinat. Dies ist mit einer normalen Ernährung nicht zu schaffen.

Um nun weiterhin den Bedarf von 1000 mg Kalzium mit der Ernährung zu decken, müsste man so viel essen: 1100 g Hüttenkäse oder 850 g Magermilch oder 750 g Joghurt oder 330 g Mozzarella oder 110 g Hartkäse oder 220 g Kerbel oder 330 g Sojabohnen oder 440 g Grünkohl oder 440 g Gartenkresse oder 880 g Brokkoli, Mangold, Spinat oder Fenchel. Auch dies ist mit einer normalen Ernährung nicht zu schaffen.

Bereits die nominale Versorgung der Bevölkerung mit Kalzium und Magnesium reicht - selbst nach den offiziellen Zahlen der Bundesorganisationen - nicht aus. Berücksichtigt man dazu noch die Bioverfügbarkeit und die Versorgungsformen, dann ist die reale Versorgung der Bevölkerung noch schlechter. Wie schlecht genau ist nicht bekannt, da diese Zahlen erstaunlicherweise nicht erhoben werden. Bekannt sind aber die Folgen dieser Mängel: Jede zweite Frau im Alter über 50 und jeder dritte Mann erkranken an Osteopenie oder Osteoporose. Das sind die Folgen einer dreißigjährigen Mangelversorgung mit Kalzium und Magnesium. Der Funktionszusammenhang zwischen Kalzium- und/oder Magnesiumdefiziten und chronischer Entzündung, chronischer Erkrankung und vorzeitiger Alterung werden wissenschaftlich diskutiert.

Bor ist ein Spurenelement und beteiligt sich neben Kalzium entscheidend an der Biochemie des Knochens. Es verhindert Osteoporose. Außerdem werden Bor Funktionen in der präventiven chronischen Entzündung und beim Prostata-Karzinom zugeschrieben. Die Verwendung von Bor in Nahrungsergänzungsmitteln in Europa und Deutschland ist schwierig, da die meisten Bor-Salze von der EU dafür nicht zugelassen sind.

Aufgabe der vorliegenden Erfindung ist es daher, eine Nahrungsergänzung zur Verfügung zu stellen, welche in der Lage ist, die durch Vitamin-D3-Mangel verursachten Störungen zu beseitigen.

Die Aufgabe wird gelöst durch die Zurverfügungstellung einer Nahrungsergänzung zur Verwendung bei der Behandlung von Vitamin-D3-Mangelerscheinungen bei einem Patienten in der Altersklasse ab 12 Jahren mit den Merkmalen des Hauptanspruchs. Vorteilhafte Ausgestaltungen sind in den abhängigen Unteransprüchen gekennzeichnet.

Offenbart wird eine Nahrungsergänzung, umfassend Kalzium, Magnesium und Bor in Kombination mit Vitamin D3, wobei Kalzium, Magnesium und Bor in Form von physiologisch verträglichen Salzen und/oder in Form von Komplexen vorliegen sowie pharmazeutisch verträgliche Hilfsstoffe und/oder Trägerstoffe.

Offenbart wird eine Nahrungsergänzung, wobei Kalzium und Magnesium in Form von physiologisch verträglichen Salzen mit organischen Säuren mit einem oder mehreren Karbonsäureresten und/oder Komplexen vorliegen. Dabei ist weiterhin bevorzugt, dass die organischen Säuren ausgewählt sind aus Essigsäure, Bernsteinsäure, Zitronensäure, Äpfelsäure, Weinsäure, Fumarsäure, Malonsäure oder Maleinsäure oder deren Mischungen.

Offenbart wird eine Nahrungsergänzung, wobei Kalzium in Form von Kalziumcitrat, Magnesium in Form eines Citratkomplexes und Bor in Form von Natriumborat vorliegt.

Offenbart wird die Verwendung einer Nahrungsergänzung, bei der Vitamin D3 einerseits, und Kalzium, Magnesium und Bor in Kombination andererseits, in jeweils getrennten Applikationsformen vorliegen. Dies dient dem Zweck, die Dosierungskombination individualisieren zu können, d.h. auf Alters-, Geschlechts und Körpergewichtsunterschiede Rücksicht nehmen zu können und somit individuell optimale Substitutionsregimes zu erzielen.

Offenbart wird ferner die Verwendung einer Nahrungsergänzung, wobei diese in oral applizierbarer Form vorliegt, wobei die oral applizierbare Form eine Kapsel, eine Tablette, ein Dragee, eine Lösung, eine Suspension und/oder eine Aufschlämmung ist.

Offenbart wird eine Nahrungsergänzung zur Verwendung bei der Behandlung von Vitamin-D3-Mangelerscheinungen, wobei die durch Vitamin-D3-Mangel verursachten Krankheiten und /oder Störungen ausgewählt sind aus Bluthochdruck (Hypertonie), Herz-Kreislauf-Erkrankungen, Herz-Kreislauf-Fehlfunktionen, Herzinsuffizienz, Vorhofflimmern, Entzündungserkrankungen des Herzens und der Niere , Arteriosklerose, Herzinfarkt, Typ-2-Diabetes, Übergewicht, metabolisches Syndrom, Fettstoffwechselstörungen, Dyslipidämie, Entzündung des Gefäß-Endothels, chronische Nierenerkrankung, Infektionsanfälligkeit, Autoimmunerkrankungen, Hashimoto, Multiple Sklerose, Psoriasis, oxidativer Stress, Demenz, neurodegenerative Erkrankungen, Schlaganfall, Krebs, Störungen des Darm-Mikrobioms, Bakterielle Infektion der Vagina, Schwangerschafts-Diabetes, Kalziummangel, Schwangerschafts-Osteoporose, hohe Infektanfälligkeit, Schwangerschaftshypertonie, Präeklampsie, Erkrankungen des Zahnfleisches, Periodontitis, Zahnwurzelentzündungen, Lebererkrankungen, intrahepatische Cholestase, Störungen des Zuckerstoffwechsels, Insulinresistenz, hoher Kalziumverlust der stillenden Mutter bei gleichzeitig unzureichender Kalzium und Vitamin D3 Versorgung des Säuglings.

Ausführungsformen der vorliegenden Erfindung ist die im Anspruch 1 definierte Nahrungsergänzung zur Verwendung wie im Anspruch 1 definiert, wobei die durch Vitamin-D3-Mangel verursachten Krankheiten und/oder Störungen ausgewählt sind aus Bluthochdruck (Hypertonie), Herz-Kreislauf-Erkrankungen, Herz-Kreislauf-Fehlfunktionen, Herzinsuffizienz, Vorhofflimmern, Entzündungserkrankungen des Herzens und der Niere, Arteriosklerose, Herzinfarkt, Typ-2-Diabetes, Übergewicht, metabolisches Syndrom, Fettstoffwechselstörungen, Dyslipidämie, Entzündung des Gefäß-Endothels, chronische Nierenerkrankung, Infektionsanfälligkeit, Autoimmunerkrankungen, Hashimoto, Multiple Sklerose, Psoriasis, oxidativer Stress, Demenz, neurodegenerative Erkrankungen, Schlaganfall, Krebs, Störungen des Darm-Mikrobioms, bakterielle Infektion der Vagina, Schwangerschafts-Diabetes, Kalziummangel, Schwangerschafts-Osteoporose, hohe Infektanfälligkeit, Schwangerschaftshypertonie, Präeklampsie, Erkrankungen des Zahnfleisches, Periodontitis, Zahnwurzelentzündungen, Lebererkrankungen, intrahepatische Cholestase, Störungen des Zuckerstoffwechsels, Insulinresistenz, hoher Kalziumverlust der stillenden Mutter bei gleichzeitig unzureichender Kalzium und Vitamin D3 Versorgung des Säuglings.

Eine Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Bluthochdruck (Hypertonie) ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung eine Herz-Kreislauf-Erkrankung ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheiten und/oder Störungen Herz-Kreislauf-Fehlfunktionen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Herzinsuffizienz ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Vorhofflimmern ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Entzündungserkrankungen des Herzens und der Niere sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Arteriosklerose ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Herzinfarkt ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Typ-2-Diabetes ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Übergewicht ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangelerscheinungen verursachte Krankheit und/oder Störung ein metabolisches Syndrom ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Fettstoffwechselstörungen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Dyslipidämie ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung eine Entzündung des Gefäß-Endothels ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung eine chronische Nierenerkrankung ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Infektionsanfälligkeit ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Autoimmunerkrankungen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Hashimoto-Thyreoiditis ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Multiple Sklerose ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Psoriasis ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangelverursachte Krankheit und/oder Störung oxidativer Stress ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Demenz ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung neurodegenerative Erkrankungen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Schlaganfall ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Krebs ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Störungen des Darm-Microbioms sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung bakterielle Infektion der Vagina sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Schwangerschafts-Diabetes ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Kalziummangel während der Schwangerschaft ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Schwangerschafts-Osteoporose ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung hohe Infektanfälligkeit während der Schwangerschaft ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Schwangerschaftshypertonie ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Präeklampsie ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Erkrankungen des Zahnfleisches sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Periodontitis ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Zahnwurzelentzündungen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Lebererkrankungen sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung intrahepatische Cholestase ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Störungen des Zuckerstoffwechsels sind.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung Insulinresistenz ist.

Eine weitere Ausführungsform der vorliegenden Erfindung ist die im Anspruch 1 definierte Nahrungsergänzung zur Verwendung wie im Anspruch 1 definiert, wobei die durch Vitamin-D3-Mangel verursachte Krankheit und/oder Störung hoher Kalziumverlust der stillenden Mutter bei gleichzeitig unzureichender Kalzium und Vitamin D3 Versorgung des Säuglings ist.

Eine weitere Ausführungsform der vorliegenden Erfindung ist die im Anspruch 1 definierte Nahrungsergänzung zur Verwendung wie im Anspruch 1 definiert, unter Verabreichung der Nahrungsergänzung gemäß einem Dosierschema, bei dem man Kalzium, Magnesium und Bor mindestens zweimal täglich und Vitamin D3 mindestens einmal wöchentlich appliziert.

Eine weitere Ausführungsform der vorliegenden Erfindung ist die im Anspruch 1 definierte Nahrungsergänzung zur Verwendung wie im Anspruch 1 definiert, unter Verabreichung der Nahrungsergänzung gemäß einem Dosierschema, bei dem man Kalzium, Magnesium und Bor mindestens zweimal täglich und Vitamin D3 mindestens einmal wöchentlich appliziert.

Eine weitere Ausführungsform der vorliegenden Erfindung ist die im Anspruch 1 definierte Nahrungsergänzung zur Verwendung wie im Anspruch 1 definiert, unter Verabreichung der Nahrungsergänzung gemäß einem Dosierschema, bei dem man Kalzium, Magnesium und Bor mindestens zweimal täglich und Vitamin D3 mindestens einmal wöchentlich appliziert.

Erfindungsgemäß ist dabei, dass die Tagesdosis für einen Erwachsenen an Kalzium mindestens 750 mg, die Tagesdosis an Magnesium mindestens 375 mg und die Tagesdosis an Bor mindestens 2 mg beträgt. Die tatsächlich zu applizieren der Tagesdosis hängt vom Alter und vom Zustand der jeweiligen zu behandelnden Person ab. Bei schwangeren oder älteren Menschen können die Tagesdosen entsprechend höher gewählt werden.

Es ist weiterhin bevorzugt, dass man Kalzium, Magnesium und Bor zweimal täglich appliziert, um eine optimale physiologische Regulation zu erzielen, welche die Halbwertszeiten der verschiedenen Stoffe in diesem komplexen System berücksichtigt.

Es ist bevorzugt, dass die Applikation an Kalzium, Magnesium und Bor morgens und abends erfolgt. Bei Schwangeren kann aber auch eine Tagesdosis von mindestens 1500 mg Kalzium, 750 mg Magnesium 4 bis 6 mg Bor vorteilhaft sein. Die Tagesdosis wird dabei vorteilhafterweise in drei Einzelgaben, morgens, mittags und abends, appliziert.

Es ist eine Verwendung offenbart, bei der das Dosierungsschema dadurch gekennzeichnet ist, dass die applizierte Dosis an Vitamin D3 zwischen bis zu 6.000 IE pro Tag und 40000 IE pro Woche beträgt.

Bevorzugt ist dabei insbesondere, dass man Vitamin D3 in einer Dosis von mindestens 400 IE täglich bis zu 40.000 IE wöchentlich appliziert. Ganz besonders bevorzugt ist hierbei, dass man die wöchentliche Dosis in bis zu drei Teildosen appliziert. Dabei wird die Applikationsmenge mit dem Ziel derart individualisiert, einen Serum-Calcidiol-Spiegel von ca. 50 ng/ml zu bewirken.

Überraschenderweise wurde gefunden, dass ein Massenverhältnis der Inhaltsstoffe der Nahrungsergänzung von 2 : 1 : 0,006 (Ca : Mg : B) besonders vorteilhaft ist. Die Nahrungsergänzung ist in der Lage, das physiologisch optimale Verhältnis von Kalzium und Magnesium im Köper rasch wiederherzustellen.

Insbesondere in Kombination mit einer Vitamin-D3-Substitution kann das optimale physiologische Verhältnis der am Vitamin-D3-Stoffwechsel beteiligten Hormone wiederhergestellt werden. Vitamin D3 sowie Kalzium, Magnesium und Bor in Kombination, stellt eine wirkungsvolle und effiziente Nahrungsergänzung zur Verfügung. Diese Nahrungsergänzung ist in einfacher Weise applizierbar und weist eine hohe Compliance durch den Patienten auf. Dieser ist durch die entsprechende Einnahme der Kombination der Nahrungsergänzung in seinem Lebensablauf nicht beeinträchtigt.

Die Herstellung der Nahrungsergänzungen folgt den allgemeinen Grundsätzen zur Herstellung von pharmazeutischen Erzeugnissen. Die Herstellung der Nahrungsergänzungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19, 241-271; Voigt, Pharmazeutische Technologie, Berlin: Ullstein Mosby 1995.

Kapseln, welche die vorgesehenen Inhaltsstoffe enthalten, können beispielsweise hergestellt werden, indem man die vorgesehenen Inhaltsstoffe mit einem inerten Träger wie Cellulose, Mikrokristalline Cellulose, Hydroxypropylmethylcellulose, Milchzucker oder Sorbit mischt und in Gelatinekapseln einkapselt.

Entsprechende Tabletten können beispielsweise durch Mischen der vorgesehenen Inhaltsstoffe mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen, Aufschlämmungen oder Suspensionen mit den vorgesehenen Inhaltstoffen der Nahrungsergänzung können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Als Lösemittel oder Suspendiermittel für die Lösungen, Aufschlämmungen oder Suspensionen können Wasser, Öle oder andere geeignete Flüssigkeiten verwendet werden. Überraschenderweise wurde gefunden, dass das Calcitriol/Calcidiol-Verhältnis von besonderer Bedeutung für die Beurteilung des Substitutionszustandes bei der Vitamin-D3-Substitution, insbesondere in Kombination mit der Kalzium/Magnesium/Bor-Substitution ist. Dieses Calcitriol/Calcidiol-Verhältnis wird vom Erfinder als "D-Status" bezeichnet. Der D-Status sollte einen Wert <1 haben, als optimal wird ein Wert von 0,5 angesehen.

Unter D-Status wird das Verhältnis von Calcitriol (pg/ml) dividiert durch Calcidiol (ng/ml) verstanden. Es wurde gefunden, dass es einen signifikanten Zusammenhang zwischen diesem Calcitriol/Calcidiol-Verhältnis nicht nur mit Parathormon, sondern auch mit dem Entzündungsmarker CRP gibt. Um so kleiner der D-Status Wert ist, also um so größer Calcidiol im Verhältnis zu Calcitriol ist, um so kleiner sind die Parathormon beziehungsweise CRP Werte. In der Extrapolation einer entsprechenden Regressionsgerade ergibt sich für einen D-Status-Wert von 0,7 ein CRP-Wert von 0, also nicht messbar. Ferner wurde gefunden, dass Calcidiol anti-entzündlich, hohe Calcitriol-Werte aber pro-entzündlich sind. Außerdem wurde gefunden, dass ein D-Status Wert < 1 optimal ist. Derartige Werte erreichten z.B. die in den Tabellen 2, 3 und 4 beschriebenen Patienten unter einer Substitution mit Kalzium/Magnesium/Bor. Während der von vielen Laboren zurzeit angegebene Referenzbereich für Calcidiol zwischen 20 ng/ml und 70 ng/ml liegt, ergibt sich nach den vom Erfinder ermittelten Daten ein optimaler Bereich von 40 ng/ml bis 60 ng/ml.

Ferner wurde überraschenderweise gefunden, dass die Nahrungsergänzung einen großen Einfluss auf das Darm-Microbiom hat. Zur Ermittlung des Zusammenhangs wurde der D-Status herangezogen und ausgewählten Organismen des Darm-Microbioms korreliert, wie dies im Beispiel 5 beschreiben ist.

Daher ist die Nahrungsergänzung in besonderer Weise geeignet das natürlich Gleichgewicht der Organismen des Darm-Microbioms wiederherzustellen.

Die nachfolgenden Beispiele erläutern die Erfindung näher, ohne den Umfang der Erfindung zu beschränken.

### Beispiel 1 (nicht erfindungsgemäß)

Herstellung einer Nahrungsergänzung enthaltende Kalzium, Magnesium und Bor in Form von Kapseln
2,566 kg Kalziumcitrat, 897,97 g Magnesiumcitratkomplex (30 %) und
14,69 g Natriumborat werden eingewogen, in einem Kubusmischer 10 Minuten lang gemischt und dann in Hartgelatinekapseln aus Hydroxypropylmethylcellulose der Größe 00 abgefüllt, wobei das Füllgewicht 806,28 mg je Kapsel beträgt.

Jede so hergestellte Wirkstoffkapsel enthält somit 125 mg Kalzium, 62,5 mg Magnesium und 0,38 mg Bor in Form der entsprechenden Ionen bezogen auf das Molekulargewicht der jeweiligen Elemente.

### Beispiel 2

Die optimale Dosierung der Nahrungsergänzung ist abhängig vom Alter und vom Zustand der zu behandelnden Personen. Erfindungsgemäß wird die Dosierung der Kombination aus Kalzium, Magnesium und Bor täglich erfolgen. Dies bedeutet, dass die erforderliche Tagesdosis im Verlaufe des Tagesablaufes einzunehmen ist. Dabei hat sich als besonders vorteilhaft herausgestellt, dass sich die Einnahme morgens und abends als besonders wirksam erwies.

In der nachfolgenden Tab. 1 sind in den entsprechenden Spalten das Alter und die jeweilige zu applizieren Menge an Vitamin D3 sowie der Kombination an Kalzium Magnesium und Bor angegeben.

Bei Kindern in einem Alter unter sechs Jahren ist eine Substitution von Kalzium, Magnesium und Bor nicht angezeigt. Jedoch sollte bei Kindern diesen Alters der Vitamin-D3-Spiegel berücksichtigt werden und gegebenenfalls eine Applikation von Vitamin D3 erfolgen.

Für die übrigen Altersklassen von Patienten ist eine Substitution von Kalzium, Magnesium und Bor mit einer entsprechenden Tagesdosis von mindestens 750 mg Kalzium, mindestens 375 mg Magnesium und mindestens 2 mg Bor angezeigt. Je nach Status des Patienten ist die zu applizieren Menge an Vitaminen D3 individuell zu wählen. Die nachfolgende Tabelle gibt hierfür entsprechende Anhaltspunkte und Vorgaben. Die Angaben für die Kalzium, Magnesium und Bor-Substitution entsprechen der Zusammensetzung der Kapsel gemäß Beispiel 1.

**Tabelle 1: Dosierung von Vitamin D3 und Kalzium / Magnesium / Bor**

| Alter (Jahre) | Vitamin D3 (IE) | Kalzium / Magnesium / Bor |
|---|---|---|
| 0 - 1 | 400 IE / Tag | Keine Substitution |
| 1 - 3 | 1000 IE / Tag | Keine Substitution |
| 3 - 6 | 1000 - 1500 IE / Tag | Keine Substitution |
| 6 - 12 | 10.000 IE / Woche | Ca 125 mg / Mg 62,5 mg./ B 0,38 mg 2 - 0 - 2 |
| 12 - 18 | 20.000 IE / Woche | Ca 125 mg / Mg 62,5 mg./ B 0,38 mg 3 - 0 - 3 |
| 18 - 60 | 20.000 - 30.000 IE / Woche | Ca 125 mg / Mg 62,5 mg./ B 0,38 mg 3 - 0 - 3 |
| 60 + | 30.000 - 40.000 IE / Woche | Ca 125 mg / Mg 62,5 mg./ B 0,38 mg 4 - 0 - 4 |
| Schwangere | 4000 - 6000 IE / Tag | Ca 125 mg / Mg 62,5 mg./ B 0,38 mg 4 - 0 - 4 bis 4 - 4 - 4 |

### Beispiel 3

Im folgenden Beispiel wird der Therapieverlauf an drei weiblichen Patienten dargestellt. Die Patientinnen waren im Jahr 1940 1986 und 1976 geboren.

Alle drei Patientinnen (Tabelle 2) klagten über Abgeschlagenheit, Antriebslosigkeit, Muskel- und Gelenkbeschwerden sowie häufige Infekte. Die 1940 geborene Patientin litt zusätzlich unter andauernden Rückenschmerzen. Eine DEXA-Messung bestätigte bei ihr eine Osteoporose. Die 1968 und 1976 geborenen Patientinnen führten auf Eigeninitiative schon eine Vitamin-D3-Substitution durch - allerdings ohne dass es jemals eine Calcidiol-Bestimmung gegeben hätte. Die 1968 geborene Patientin nahm 2000 IE Vitamin D3 pro Tag, die 1976 geborene Patientin 1000 IE (Tabelle 2). Während die Patientin, die 1000 IE Vitamin D3 einnahm, die Mindestgrenze für eine als ausreichend angesehene Versorgung nicht erreichte (27,6 ng/ml), erreichte die, die 2000 IE Vitamin D3 täglich einnahm sie knapp (30 ng/ml). Die ältere der beiden (1968 geboren) litt zudem seit längerer Zeit unter sehr schmerzhaften Muskelkrämpfen, vor allem nachts. Deshalb nahm sie schon lange Magnesium ein: 3 x 300 mg Magnesium-Citrat, ein Monopräparat. Die Beschwerden aber blieben. Dies erklärt sich insofern, als dass auch ein intrazellulärer Kalzium-Überschüsse Muskelkrämpfe auslösen kann. Kalzium ist an der Muskelkontraktion entscheidend beteiligt. Außerdem kann die Zelle Magnesium in einem solchen Fall nicht aufnehmen. Die 1976 (2000 IE Vitamin D3 !) und 1940 (kein Vitamin D3) geborenen Frauen litten zudem an einem sekundären Hyperparathyreoidismus, während die 1968 geborene einen tiefen Parathormon Spiegel aufwies. Das heißt, die Parathormon-Produktion in der Nebenschilddrüse war wegen des niedrigen Serum-Ca²⁺/Mg²⁺ sehr hochgeregelt. Das sich dieser sekundäre Hyperparathyreoidismus bei der 1968 geborenen Patientin nicht zeigte, war eine Folge ihrer hohen Magnesium-Substitution, die aber, hinsichtlich des beabsichtigten Ziels (Aufheben von Muskelkrämpfen) erfolglos geblieben war. Bei allen drei Patientinnen wurde ein sehr hohes und damit pro-entzündliches Calcitriol, das zudem eine fortschreitende osteoporotische Stoffwechsellage schafft, festgestellt. Bei der ältesten Patientin hatte das inzwischen auch zu einer manifesten Osteoporose geführt. Trotz der ungezielt dosierten Substitution mit Vitamin D3 (2000 IE Vitamin D3 pro Tag) erreichte die 1968 geborene Patientin nur die untere Grenze des Calcidiol-Referenzbereichs (30 ng/ml). Und die 1976 geborene Frau lag mit ihrer Eigensubstitution (1000 IE Vitamin D3 pro Tag) sogar immer noch im Mangelbereich. Daraus folgt auch, dass die von offiziellen Stellen, zum Beispiel von der DGE verschlagene "ausreichende" Vitamin D3 Substitution von 800IE Vitamin D3 pro Tag völlig unzureichend ist. Bei der 1940 geborenen Patientin stellte ich einen schweren Calcidiol-Mangel fest. Der Calcitriol/Calcidiol Quotient war in allen drei Patientinnen sehr ungünstig und stand für ein zu hohes Calcitriol, dass sowohl die stille Entzündung, als auch die osteoporotische Knochenstoffwechsel Lage begünstigte. In den 1976 und 1940 geborenen Patientinnen erfüllte der Parathormon Wert sogar die schulmedizinischen Kriterien eines sekundären Hyperparathyreoidismus. Dieser wird als Folge eines a) Vitamin-D3-Mangels, b) einer Nierenunterfunktion angesehen. Parathormon wird in der Schulmedizin fast nur bei Patienten mit Niereninsuffizienz gemessen. Betrachtet man den Calcidiol-Wert der 1976 geborenen Patientin erscheint es unwahrscheinlich, dass dieser Wert durch die übliche Labordiagnostik entdeckt worden wäre, weil der Wert nur minimal vom "unteren" Referenzbereich abweicht (einige Labore betrachten einen Wert von 20 ng/ml Calcidiol als ausreichend), und weil Parathormon bei "Nierengesunden" äußerst selten gemessen wird. Ein hohes Parathormon begünstigt übrigens an und für sich auch ein erhöhtes Krebs und Demenz Risiko. Diese drei Beispiele unterstreichen folgende Punkte:
1. Eine ungezielte Substitution ohne Messung von zumindest Calcidiol ist nicht zielführend. Sie beseitigt die Unterversorgung meistens nicht.
2. Auch bei der Substitution von Vitamin D3 kann ohne gleichzeitige Kalzium-Magnesium-Substitution keine Stoffwechsel-Normalisierung erreicht werden.
3. Auch eine alleinige Substitution mit Magnesium wegen Muskelkrämpfen, erbringt keine Normalisierung des Vitamin-D3-Systems (Calcidiol/Calcitriol). Denn der Kalzium-Mangel wird nicht behoben.
4. Das Calcitriol-Calcidiol-Verhältnis kann nicht allein durch eine Vitamin-D3-Substitution optimiert werden - selbst dann nicht, wenn es besser sein sollte als bei der 1940 geborenen Patientin, deren Vitamin-D3-Stoffwechsel sich im freien Fall befand.

**Tabelle 2: Laborparameter der untersuchten Patientinnen.**

| Parameter | Patientin, 1968 geb. 1) 2) | Patientin, 1976 geb. 1) | Patientin, 1940 geb. | Referenzbereich |
|---|---|---|---|---|
| Parathormon (pg/ml) | 25,5 | **65,7** | **77,3** | 15 - 65 (pg/ml) |
| Calcitriol (pg/ml) | **73,9** | 62,0 | **81,1** | 20 - 63 (pg/ml) |
| Calcidiol (ng/ml) | 30,0 | **27,2** | **11,5** | 30 - 70 (ng/ml) |
| Calcitriol/Calcidiol-Verhältnis | **2,46** | **2,28** | **7,05** | nicht definiert |

| | | | | |
|---|---|---|---|---|
| 1) Patientinnen substituierten Vitamin D3 auf eigene Initiative. 2) Patientin substituierte Magnesium (3x300mg pro Tag). Krankhafte Werte sind in Fettdruck dargestellt. | | | | |

Am Beispiel der 1968 geborenen Patientin soll nun der Therapieverlauf beschreiben werden. Sie erhielt zum einen 2 mal pro Woche 10.000 IE Vitamin D3 (Mittwoch und Sonntag zum Essen). Zusätzlich nahm sie 1000 mg Kalzium, 500 mg Magnesium und 3 mg Bor ein (Tabelle 3). Aus den in Tabelle 3 wiedergegebenen Entzündungsparametern erkennt man, dass die Patientin anfangs eine erhebliche chronische Entzündung hatte, die in diesem Fall auch symptomatisch war (Muskelschmerzen, Rückenschmerzen, chronische Müdigkeit, kein erholsamer Schlaf, morgens immer unausgeruht und erschöpft). Gleichzeitig wurde in der Erst-Bestimmung ein sehr niedriger ATP-Wert ermittelt- die biochemische Erklärung für die Antriebslosigkeit. Dieser Wert zeigt die schwere Beeinträchtigung des zellulären (mitochondrialen) Energiestoffwechsels. Nach dem Absetzen der Magnesium-Monotherapie, der Erhöhung der Vitamin-D3-Versorgung und der Umstellung auf eine kombinierte Kalzium-Magnesium-Bor-Substitution normalisierten sich die Entzündungsparameter nicht nur. Die Patientin erreichte hier sogar optimale Werte. Die Energieproduktion verdreifachte sich, die beklagte Symptomatik verschwand vollständig. Bei der ersten Nachmessung sechs Monate nach Therapiebeginn war das Verhältnis von Calcitriol zu Calcidiol optimal und blieb so im Verlauf. Ebenso sind die Calcidiol-Werte nun äußerst stabil. Der Anstieg des Parathormons erklärt sich durch die Reduktion der täglichen Magnesium-Substitution vom 900 auf 500 mg/Tag. Hier könnte man sagen, dass diese Patientin vielleicht eine zusätzliche höhere Magnesium Substitution aufgrund ihrer Lebensbedingungen gebrauchen könnte. Parathormon-Werte von 48,6 pg/ml (28.01.13) und 49,4 pg/ml (17.04.14) sind zwar im Deutschen Referenzbereich, für optimale Werte aber zu hoch.

**Tabelle 3: Therapieverlauf bei einer Patientin**

| Parameter | | | | | Referenzwerte |
|---|---|---|---|---|---|
| Patientin, geb. 1968 - Verlauf der Vitamin-D3-Kalzium-Magnesium-Bor-Therapie über 2 Jahre | | | | | |
| Datum | 11.04.2012 | 28.01.2013 | 28.05.2013 | 17.04.2014 | |
| Parathormon | 25,5 | 48,6 | 37,1 | 49,4 | 15 - 65 pg/ml |
| Calcidiol | 30,0 | 50,7 | 53,1 | 52,1 | 30 - 70 ng/ml |
| Calcitriol | **73,9** | 38,0 | 15,0 | 26,0 | 20 - 63 pg/ml |
| Calcitriol / Calcidiol Verhältnis | **2,46** | 0,75 | 0,28 | 0,5 | nicht definiert |
| | | | | | Soll: ≤ 1 |
| | | | | | Ideal um 0,5 |
| CRPs | **8,6** | 0,49 | ND | 0,41 | < 3.0 mg/L |
| | | | | | Ideal < 0,3 |
| IL6 | **4,1** | **2,9** | ND | < 2,0 | < 2.0 pg/ml |
| IL8 intrazellulär | **250** | 133 | ND | 115 | < 150 pg/ml |
| ATP | **1,49** | ND | 2,63 | 4,24 | >2 µM |

| | | | | | |
|---|---|---|---|---|---|
| Krankhafte Werte sind in Fettdruck dargestellt. | | | | | |

### Beispiel 4

In Tabelle 4 ist das Beispiel eines Mannes dargestellt, der unter gleichbleibender Substitution mit Vitamin D3 (zweimal pro Woche, Mittwoch und Sonntag je 20'000IE zum Essen) unterschiedliche Kalzium/Magnesium Substitutionen durchführte, und zwischendurch einmal für 14 Tage die Kalzium oder Kalzium/Magnesium Substitution unterbrach. Aus dem Beispiel ist ersichtlich, dass:
1. Nur eine Substitution mit Kalzium/Magnesium/ Bor zu einem optimalen Calcitriol/Calcidiol Verhältnis führte.
2. Die alleinige Substitution von Kalzium, selbst in einer Dosis von 2000 mg pro Tag nicht in der Lage war, die Calcitriol Spiegel optimal zu senken.
3. Die Unterbrechung der Kalzium-Magnesium-Bor Substitution zu einer Verdopplung des Plasma Parathormon Spiegels und zu einer Verfünffachung des Calcitriol-Serumspiegels führte.
4. Eine alleinige Substitution mit Kalzium weder den Parathormon-Wert noch den Calcitriol-Wert in die vorherige optimale Situation zurückführen konnte.
5. Die Erhöhung der Kalzium Substitution auf 2000 mg pro Tag (im Vergleich zu 1000 mg täglich) nur zu einer geringfügigen weiteren Senkung von Parathormon führte.

**Tabelle 4: Unterschiedliche Substitutionsregimes mit Kalzium allein oder Kalzium/Magnesium/Bor (gemäß Beispiel 1)**

| Parameter | nach 8 Wochen Substitution | Nur Vitamin D3 Substitution für 2 Wochen | Vitamin D3 mit 1000 mg Kalzium nach 3 Wochen | Vitamin D3 mit 2000 mg Kalzium nach 3 Wochen | Referenzwert (Standard) |
|---|---|---|---|---|---|
| Parathormon | 19,7 | 37,2 | 30,1 | 25,7 | 15-65 pg/ml |
| Calcitriol | 28,7 | **153** | **130** | **112,8** | 23 - 65 pg/ml |
| Calcidiol | 55,4 | 57,9 | 58,8 | 50,9 | 30-70 ng/ml |
| Calcitriol/Calcidiol Verhältnis | 0,52 | **2,64** | **2,21** | **2,21** | nicht definiert |
| Ca/Mg Substitution | Beispiel 1 | Keine Ca / Mg Substitution | Calcium Sandoz Fortissimum® 1 - 0 - 0 | Calcium Sandoz Fortissimum® 1 - 0 - 1 | |

Auch dieses Beispiel zeigt, dass eine alleinige Substitution mit Vitamin D3 nicht sinnvoll ist, weil sie, gerade bei einem dadurch erreichten höheren Calcidiol-Spiegel, zu krankhaft erhöhten Calcitriol-Spiegeln führen kann. Dies gilt insbesondere dann, wenn zum Beispiel eine säurebetonte Ernährung vorliegt. Die alleinige, selbst hoch dosierte Ergänzung der Vitamin D3 Substitution mit Kalzium normalisiert diese Hypercalcitriolämie nicht ausreichend. Diese "Risiko-Konstellationen" werden schon deshalb nicht erkannt, weil Calcitriol und Parathormon nicht zusammen mit Calcidiol messen werden. Das bedeutet für den Patienten, der zwar vielleicht wegen eines diagnostizierten "Vitamin D3 Mangels" (tiefes Calcidiol), eine wie auch immer geartete Vitamin D3 Substitution empfohlen bekommt (meist zu tief), dass sich seine gesundheitliche Problematik sogar noch verschlechtern kann, weil z.B. durch ein weiter erhöhtes Calcitriol die chronische Entzündung noch weiter "angeheizt" wird (Vergleichend hierzu die 1968 geborene Patientin, Tabelle 2 und 3). Da die Entzündungsparameter IL6 und IL8 fast nie bestimmt werden, und der relativ häufig gemessene Entzündungsparameter CRP nie mit einer Vitamin D3 / Kalzium / Magnesium Störung in Verbindung gebracht wird, bleiben die Ursachen der "stillen" chronischen Entzündung im Regelfall im Dunkeln. Die chronische Entzündung bleibt, und kann auch (auch bei Vitamin D3 Monosubstitution) weiterhin bestehen bleiben.

Die Beispiele in den Tabellen 2 bis 4 zeigen zudem die zusätzliche Wirkung der begleitenden Substitution mit Bor. Bor verschiebt das Calcidiol/Calcitriol-Verhältnis zugunsten von Calcidiol. Nur mit der kombinierten Substitution von Kalzium, Magnesium und Bor scheint es möglich zu sein, ein optimales, das heißt anti-entzündliches Calcitriol/Calcidiol-Verhältnis (D-Status) zu erreichen.

### Beispiel 5 (nicht erfindungsgemäß)

Als Microbiom bezeichnet man die Gesamtheit aller Bakterien, die im (Mund)-Magen-Darm Trakt leben. Das Microbiom eines gesunden Erwachsenen hat in etwa die Masse von 1 - 1,5 kg und besteht aus ca. 500 verschiedenen Bakterien. Insgesamt sind für das menschliche Microbiom ca. 1200 verschiedene Bakterien-Gattungen und 36000 verschiedene Bakterien bis heute nachgewiesen worden. Das menschliche Microbiom ist so individuell wie ein Fingerabdruck.

In der Untersuchung an 50 Erwachsenen wurde der Zusammenhang zwischen Calcitriol und dem D-Status mit CRPhs (also systemischer Entzündung) ermittelt, wobei auch zwei nur molekularbiologisch untersuchbare Darmbakterien quantitativ bestimmt wurden: Fäcalibakterium Prausnitzii und toxische Clostridien des Clusters I. Fäcalibacterium Prausnitzii ist eines der wichtigsten Bakterien für die Aufrechterhaltung der Microbiom-Homöostase. Toxische Clostridien Cluster I sind potentiell krankheitserregend. Sie können schwere Infekte, bei älteren Menschen z.B. auch Lungenentzündung vermitteln. Nur wenige der 50 untersuchten Personen wiesen ein "normales" Stuhlprofil für diese beiden Bakterien auf. Eine Korrelation von beiden Bakterien mit dem D-Status dieser Personen zeigte eine signifikante Beziehung zwischen der normalen und der pathologischen Darmflora und dem D-Status auf. Die Ergebnisse sind in Tabelle 5 dargestellt.

**Tabelle 5: Der D-Status**

| Miteinander korrelierte Parameter | D-Status-Wert |
|---|---|
| Parathormon gegen Status (für einen oberen Parathormon Referenzwert von 40 pg/ml) ¹⁾ | 1,44 |
| CRPhs gegen D-Status (für ein CRPhs von 0) ²⁾ | 0,75 |
| Fäcalibacterium Prausnitzii (für 2x10¹⁰ Bakterien/g Stuhl) ³⁾ | 0,6 |
| Toxische Clostridien Cluster I (für 5x10⁸ Bakterien/g Stuhl) ⁴⁾ | 0,15 |
| Mittelwert | 0,74 |
| Referenzbereich | 0,5 - 1,0 |

| | |
|---|---|
| 1) Aus den Untersuchungen ergibt sich für einen Calcidiol Wert von 30 ng/ml ein oberer Parathormon-Plasmaspiegel von 40 pg/ml. 2) Für den "Zustand" minimaler chronischen (stiller) Entzündung ist ein CRPhs Wert unterhalb der Messwertgrenze anzustreben. 3) Eine Anzahl von Fäcalibacterium Prausnitzii die einen gesunden Darm Flora Status wiedergibt. 4) Anzahl von toxischen Clostridien Cluster I die im Darm nicht überschritten werden soll. | |

## Patentansprüche

1. Nahrungsergänzung, umfassend Kalzium, Magnesium und Bor in Kombination mit Vitamin D3, wobei Kalzium, Magnesium und Bor in Form von physiologisch verträglichen Salzen und/oder in Form von Komplexen vorliegen sowie pharmazeutisch verträgliche Hilfsstoffe und/oder Trägerstoffe, zur Verwendung bei der Behandlung von Vitamin-D3-Mangelerscheinungen bei einem Patienten in der Altersklasse ab 12 Jahre,
unter Verabreichung der Nahrungsergänzung gemäß einem Dosierschema, bei dem man dem Patienten in der Altersklasse ab 12 Jahre mindestens 750 mg Kalzium, mindestens 375 mg Magnesium und mindestens 2 mg Bor täglich appliziert,
und wobei die applizierte Dosis an Vitamin D3 pro Woche bei einem Jugendlichen im Alter von 12 - 18 Jahren 20.000 IE, bei einem Erwachsenen im Alter von 18 - 60 Jahren zwischen 20.000 - 30.000 IE und bei einem Erwachsenen im Alter von über 60 Jahren zwischen 30.000 - 40.000 IE beträgt.

2. Nahrungsergänzung, zur Verwendung gemäß Anspruch 1, wobei Kalzium und Magnesium in Form von physiologisch verträglichen Salzen mit organischen Säuren mit einem oder mehreren Karbonsäureresten und/oder Komplexen vorliegen.

3. Nahrungsergänzung, zur Verwendung gemäß Anspruch 2, **dadurch gekennzeichnet, dass** die organischen Säuren ausgewählt sind aus Essigsäure, Bernsteinsäure, Zitronensäure, Äpfelsäure, Weinsäure, Fumarsäure, Malonsäure oder Maleinsäure oder deren Mischungen.

4. Nahrungsergänzung, zur Verwendung gemäß einem der voranstehenden Ansprüche, **dadurch gekennzeichnet, dass** Kalzium in Form von Kalziumcitrat, Magnesium in Form eines Citratkomplexes und Bor in Form von Natriumborat vorliegt.

5. Nahrungsergänzung, zur Verwendung gemäß einem der voranstehenden Ansprüche, **dadurch gekennzeichnet, dass** Vitamin D3 einerseits, und Kalzium, Magnesium und Bor in Kombination andererseits, in jeweils getrennten Applikationsformen vorliegen.

6. Nahrungsergänzung, zur Verwendung gemäß einem der voranstehenden Ansprüche, **dadurch gekennzeichnet, dass** diese in oral applizierbarer Form vorliegt, wobei die oral applizierbare Form eine Kapsel, eine Tablette, ein Dragee, eine Lösung, eine Suspension und/oder eine Aufschlämmung ist.

7. Nahrungsergänzung, zur Verwendung gemäß einem der voranstehenden Ansprüche, wobei die durch Vitamin-D3-Mangel verursachten Krankheiten und/oder Störungen ausgewählt sind aus Bluthochdruck (Hypertonie), Herz-Kreislauf-Erkrankungen, Herz-Kreislauf-Fehlfunktionen, Herzinsuffizienz, Vorhofflimmern, Entzündungserkrankungen des Herzens und der Niere, Arteriosklerose, Herzinfarkt, Typ-2-Diabetes, Übergewicht, metabolisches Syndrom, Fettstoffwechselstörungen, Dyslipidämie, Entzündung des Gefäß-Endothels, chronische Nierenerkrankung, Infektionsanfälligkeit, Autoimmunerkrankungen, Hashimoto, Multiple Sklerose, Psoriasis, oxidativer Stress, Demenz, neurodegenerative Erkrankungen, Schlaganfall, Krebs, Störungen des Darm-Mikrobioms, Bakterielle Infektion der Vagina, Schwangerschafts-Diabetes, Kalziummangel, Schwangerschafts-Osteoporose, hohe Infektanfälligkeit, Schwangerschaftshypertonie, Präeklampsie, Erkrankungen des Zahnfleisches, Periodontitis, Zahnwurzelentzündungen, Lebererkrankungen, intrahepatische Cholestase, Störungen des Zuckerstoffwechsels, Insulinresistenz, hoher Kalziumverlust der stillenden Mutter bei gleichzeitig unzureichender Kalzium und Vitamin D3 Versorgung des Säuglings.

8. Nahrungsergänzung, zur Verwendung gemäß einem der voranstehenden Ansprüche, **dadurch gekennzeichnet, dass** man Kalzium, Magnesium und Bor zweimal täglich appliziert, um die Tagesdosis zu erzielen.

9. Nahrungsergänzung, zur Verwendung gemäß Anspruch 8, **dadurch gekennzeichnet, dass** die Applikation an Kalzium, Magnesium und Bor morgens und abends erfolgt.

10. Nahrungsergänzung, zur Verwendung gemäß einem der vorangehenden Ansprüche, **dadurch gekennzeichnet, dass** man die wöchentliche Dosis Vitamin D3 in bis zu drei Teildosen appliziert.

## Claims

1. Nutritional supplement, comprising calcium, magnesium and boron in combination with vitamin D3, wherein calcium, magnesium and boron are present in form of physiological tolerable salts and/or in form of complexes, as well as pharmaceutical acceptable excipients and/or vehicles, for use in the treatment of vitamin D3 deficiency symptoms in a patient in the age group of from 12 years on,
by administering of the nutritional supplement according to a dosing regime, wherein the patient in the age group of from 12 years on at least 750 mg Calcium, at least 375 mg magnesium and at least 2 mg boron are administered daily,
and wherein the administered dose of vitamin D3 per week in a juvenile in the age of 12 - 18 years is 20,000 IU, in an adult in the age of 18 - 60 years is between 20,000 - 30,000 IU, and in an adult in the age of more than 60 years is between 30,000 - 40,000 IU.

2. Nutritional supplement, for use according to claim 1, wherein calcium and magnesium are present in form of physiological tolerable salts with organic acids with one or more carboxylic acid esters and/or complexes.

3. Nutritional supplement, for use according to claim 2, **characterized in that** the organic acids are selected from acetic acid, succinic acid, citric acid, malic acid, tartaric acid, fumaric acid, malonic acid or maleic acid or the mixtures thereof.

4. Nutritional supplement, for use according to any of the preceding claims, **characterized in that** calcium is present in form of calcium citrate, magnesium in form of a citrate complex, and boron in form of sodium borate.

5. Nutritional supplement, for use according to any of the preceding claims, **characterized in that** on one hand vitamin D3 and calcium, magnesium and boron in combination on the other hand are each present in separate forms for application.

6. Nutritional supplement for use according to any of the preceding claims, **characterized in that** it is present in orally applicable form, whereby the oral applicable form is a capsule, a tablet, a dragee, a solution, a suspension and/or a slurry.

7. Nutritional supplement, for use according to any of the preceding claims, **characterized in that** the disease and/or disorders caused by vitamin D3 deficiency are selected from high blood pressure (hypertension), cardiovascular diseases, cardiovascular malfunctions, cardiac insufficiencies, atrial fibrillation, inflammatory diseases of the heart and the kidney, arteriosclerosis, cardiac infarction, type 2 diabetes, overweight, metabolic syndrome, lipometabolic disorders, dyslipidemia, inflammation of the vascular endothelium, chronic kidney disease, proneness to infectious diseases, autoimmune diseases, Hashimoto's disease, multiple sclerosis, psoriasis, oxidative stress, dementia, neurodegenerative diseases, stroke, cancer, disorders of the intestinal microbiome, bacterial infections of the vagina, gestational diabetes, calcium deficiency, pregnancy-associated osteoporosis, high proneness to infections, pregnancy-associated hypertonia, preeclampsia, diseases of the gums, periodontitis, inflammations of the dental root, liver diseases, intrahepatic cholestasis, disorders of the glycometabolism, insulin resistance, high loss of calcium of the breastfeeding mother with concomitant insufficient calcium and vitamin D3 supply of the suckling.

8. Nutritional supplement, for use according to any of the preceding claims, **characterized in that** one applies calcium, magnesium and boron twice a day in order to reach the daily dose.

9. Nutritional supplement, for use according to claim 8, **characterized in that** the application of calcium, magnesium and boron takes place in the morning and in the evening.

10. Nutritional supplement, for use according to any of the preceding claims, **characterized in that** one applies the weekly dose of vitamin D3 in up to three partial doses.

## Revendications

1. Complément alimentaire, contenant du calcium, du magnésium et du bore, en association avec de la vitamine D3, dans lequel le calcium, le magnésium et le bore sont sous forme de sels physiologiquement acceptables et/ou sous forme de complexes ainsi que des adjuvants et/ou supports pharmaceutiquement acceptables, pour l'utilisation dans le traitement des symptômes de carence en vitamine D3 chez un patient dans une classe d'âge à partir de 12 ans,
en administrant le complément alimentaire à une certaine posologie, selon laquelle on administre au patient dans une classe d'âge à partir de 12 ans au moins 750 mg de calcium, au moins 375 mg de magnésium et au moins 2 mg de bore quotidiennement,
et la dose administrée de vitamine D3 chez un adolescent âgé de 12 à 18 ans étant de 20.000 U.I. par semaine, chez un adulte âgé de 18 à 60 ans entre 20.000 et 30.000 U.I. par semaine et chez un adulte âgé de plus de 60 ans entre 30.000 et 40.000 U.I. par semaine.

2. Complément alimentaire pour l'utilisation selon la revendication 1, dans lequel le calcium et le magnésium sont sous forme de sels physiologiquement acceptables avec des acides organiques comprenant un ou plusieurs esters d'acide carboxylique et/ou de complexes.

3. Complément alimentaire pour l'utilisation selon la revendication 2, **caractérisé en ce que** les acides organiques sont sélectionnés parmi l'acide acétique, l'acide succinique, l'acide citrique, l'acide malique, l'acide tartrique, l'acide fumarique, l'acide malonique ou l'acide maléique ou leurs mélanges.

4. Complément alimentaire pour l'utilisation selon l'une des revendications précédentes, **caractérisé en ce que** le calcium est sous forme de citrate de calcium, le magnésium est sous forme d'un complexe de citrate et le bore est sous forme de borate de sodium.

5. Complément alimentaire pour l'utilisation selon l'une des revendications précédentes, **caractérisé en ce que** la vitamine D3 d'une part et le calcium, le magnésium et le bore en combinaison d'autre part se présentent dans des formes d'administration respectivement séparées.

6. Complément alimentaire pour l'utilisation selon l'une des revendications précédentes, **caractérisé en ce que** celui-ci se présente sous une forme d'administration par voie orale, dans lequel la forme d'administration par voie orale est une capsule, un comprimé, une dragée, une solution, une suspension et/ou une suspension épaisse.

7. Complément alimentaire pour l'utilisation selon l'une des revendications précédentes, dans lequel les maladies et/ou troubles causés par une carence en vitamine D3 sont sélectionnés parmi la pression artérielle élevée (hypertension), les maladies cardiovasculaires, les dysfonctionnements cardiovasculaires, l'insuffisance cardiaque, la fibrillation auriculaire, les maladies inflammatoires du cœur et du rein, l'artériosclérose, l'infarctus du myocarde, le diabète de type II, la surcharge pondérale, le syndrome métabolique, les troubles du métabolisme des lipides, la dyslipidémie, l'inflammation de l'endothélium vasculaire, la maladie rénale chronique, la prédisposition aux infections, les maladies auto-immunes, la thyroïdite de Hashimoto, la sclérose en plaques, le psoriasis, le stress oxydant, la démence, les maladies neurodégénératives, l'accident vasculaire cérébral, le cancer, les troubles du microbiote intestinal, l'infection bactérienne du vagin, le diabète de grossesse, la carence en calcium, l'ostéoporose de grossesse, la prédisposition accrue aux infections, l'hypertension de grossesse, la pré-éclampsie, les affections des gencives, la parodontite, les inflammations de la racine dentaire, les maladies du foie, la cholestase intra-hépatique, les troubles du métabolisme du sucre, la résistance à l'insuline, la perte en calcium accrue chez la femme qui allaite avec un apport insuffisant en calcium et en vitamine D3 pour le nourrisson.

8. Complément alimentaire pour l'utilisation selon l'une des revendications précédentes, **caractérisé en ce qu'**on administre du calcium, du magnésium et du bore deux fois par jour pour obtenir la dose quotidienne.

9. Complément alimentaire pour l'utilisation selon la revendication 8, **caractérisé en ce que** l'administration du calcium, du magnésium et du bore s'effectue le matin et le soir.

10. Complément alimentaire pour l'utilisation selon l'une des revendications précédentes, **caractérisé en ce qu'**on administre la dose hebdomadaire de vitamine D3 en trois doses partielles au maximum.
